# EUROPEAN PATENT APPLICATION

(11) **EP 4 480 508 A1**
(43) Date of publication of application: **25.12.2024**
(21) Application number: 24173767.5
(22) Date of filing: 02.05.2024
(51) Int. Cl.: A61L 27/04, A61F 2/30, A61L 27/06, A61L 27/10, A61L 27/16, A61L 27/18, A61L 27/30, A61L 27/50

(54) **BIOCOMPATIBLE PROTECTIVE COATING ON A MEDICAL IMPLANT COMPONENT AND PROCESS OF MAKING THE SAME**

(30) Priority: 21.06.2023 US 202363522270 P
(71) Applicant: Innojet Technology Co., Ltd., Taoyuan City 320017 (TW)
(72) Inventor: CHANG, Jen-Hsien, 320017 TAOYUAN CITY (TW); TANG, Wei-Cheng, 320017 TAOYUAN CITY (TW); TSAI, Yu-Yen, 320017 TAOYUAN CITY (TW)
(74) Representative: Vossius & Partner Patentanwälte Rechtsanwälte mbB

(57) **Abstract**

The present disclosure relates to medical implant components comprising a biocompatible protective coating layer (BPCL) and a process of making the BPCL and medical implant components.

## Description

### TECHNICAL FIELD OF THE INVENTION

The present disclosure relates to medical implant components comprising a biocompatible protective coating layer (BPCL), preferably silicon nitride (SiNₓ) layer, and a process of making the BPCL and medical implant components.

### BACKGROUND OF THE INVENTION

Medical implant components may be used in various clinical and healthcare applications in order to provide benefits for physicians, surgeons, registered professional nurses and patients during or after medical treatments. Thanks to developments in material science and medical sciences, the prognosis of patients in need of surgery or medical implants is becoming better and better. For example, artificial joints may improve motor functions of subjects or patients suffering from age-related conditions or diseases and may prolong the duration of body parts of the subjects or patients.

To enhance the performance of medical implants, coatings may be applied on the surface thereon. However, existing coated medical implants may still exhibit certain defects or disadvantages such as insufficient biocompatibility, durability or strength. In addition, costs of the existing processes for preparing the coated medical implants may still be high, and applicability of the processes may be limited in view of the materials of the medical implants.

Hence, there is still a need to develop novel and cost-effective medical implants and processes for preparing the same.

### SUMMARY OF THE INVENTION

The present disclosure thus relates to a medical implant component, comprising:
(a) a biocompatible substrate, and
(b) a biocompatible protective coating layer (BPCL) above or on the substrate;

wherein the biocompatible substrate is made from a material or materials selected from the group consisting of a biocompatible polymeric material, a biocompatible metal or alloy and a biocompatible ceramic, and
wherein the biocompatible protective coating layer (BPCL) has a porosity of less than 1.5%, preferably 1% or less.

The present disclosure also relates to a process of making a medical implant component described herein, comprising the following steps:
(i) providing a biocompatible substrate in a deposition chamber;
(ii) reducing the pressure in the deposition chamber to less than 0.75 Torr; and
(iii) depositing a material of a biocompatible protective coating layer to form the biocompatible protective layer above or on the substrate.

In one aspect, the present disclosure provides a medical implant component comprising a biocompatible substrate, a buffer layer on the substrate and a biocompatible protective coating layer on the buffer layer, i.e., the buffer layer is located between the biocompatible substrate and the biocompatible protective coating layer, wherein the buffer layer is made from oxide, nitride or carbide of any of the following elements: silicon (Si), aluminum (Al), zirconium (Zr) and boron (B).

In one aspect, the present disclosure provides a process of making a medical implant component described herein, comprising the following steps:
(i) providing a biocompatible substrate in a deposition chamber;
(ii) reducing the pressure in the deposition chamber to less than 0.75 Torr;
(ii') depositing a buffer layer material selected from the group consisting of oxide, nitride or carbide of any of the following elements: silicon (Si), aluminum (Al), zirconium (Zr) and boron (B) to form a buffer layer on the substrate; and
(iii') depositing a material of a biocompatible protective coating layer to form the biocompatible protective coating layer on the buffer layer.

In one aspect, the present disclosure provides the medical implant component as a part or the entirety of an artificial joint, an insert associated with an artificial joint or a temporary anchorage device.

In one aspect, the present disclosure provides a medical implant component, characterized in that it comprises a biocompatible protective coating layer (BPCL) and optionally a buffer layer as described herein.

In one embodiment, the biocompatible protective coating layer has a thickness of 0.5 µm to 70 µm, preferably 5 to 30 µm.

In any preceding embodiments, the biocompatible protective coating layer (BPCL) has a hardness (Vickers hardness) of at least 800 HV, preferably up to 900 HV, more preferably up to 1200 HV, most preferably up to 1400 HV.

In any preceding embodiments, the biocompatible protective coating layer (BPCL) has a surface roughness of less than 0.25 µm, preferably of 0.2 µm or less.

In any preceding embodiments, the stoichiometry change in the atoms of materials of biocompatible protective coating layer (BPCL) are smaller than 20%.

In any preceding embodiments, the biocompatible protective coating layer (BPCL) is formed from particles having D50 ranging from 0.1 µm to 10 µm, preferably ranging from 0.5 µm to 3 µm.

In any preceding embodiments, the coefficient of kinetic friction between the medical implant component and the other corresponding medical component (such as a pad or insert) ranges from 0.07 to 0.7, preferably 0.08 to 0.65, more preferably 0.1 to 0.55.

In any preceding embodiments, the biocompatible polymeric material is selected from the group consisting of polytetrafluoroethene (PTFE), polyetheretherketone (PEEK), polyethylene (PE), polyurethane (PU) and polyvinylchloride (PVC).

In any preceding embodiments, the PE is low-density polyethylene (LDPE), high-density polyethylene (HDPE) or ultra-high molecular weight polyethylene (UHMWPE).

In any preceding embodiments, the biocompatible polymeric material has a hardness of at least 400 HV (Vickers hardness), preferably up to 600 HV, more preferably up to 1000 HV; or at least a Shore D of 50 (Shore hardness), preferably at least a Shore D of 55, more preferably at least a Shore D of 60, most preferably at least a Shore D of 65.

In any preceding embodiments, the biocompatible metal or alloy is selected from the group consisting of titanium (Ti) or an alloy thereof, zirconium (Zr) or an alloy thereof, tantalum (Ta) or an alloy thereof, niobium (Nb) or an alloy thereof, stainless steel, cobalt-chromium-molybdenum (Co-Cr-Mo) alloy, and Ti-6Al-4V alloy.

In any preceding embodiments, the biocompatible ceramic is selected from the group consisting of oxide, carbide, nitride, or nitro-carbide of any of the following elements: silicon (Si), titanium (Ti), tantalum (Ta), tungsten (W), zirconium (Zr), niobium (Nb), chromium (Cr) and aluminum (Al).

In any preceding embodiments, the buffer layer has a thickness of 0.5 µm to 10 µm, preferably at least 1µm.

In any preceding embodiments, the adhesion between the buffer layer and the substrate is at least 18 Mpa, preferably at least 20 MPa, more preferably at least 25 MPa, most preferably at least 30 MPa. The adhesion may be measured by Scratch method, Rockwell C tests or ASTM-C1624.

In any preceding embodiments, the deposition of the buffer layer is achieved via AD, physical vapor deposition (PVD) or chemical vapor deposition (CVD).

In any preceding embodiments, the AD is carried out with a carrier gas selected from the group consisting of N₂, O₂, Ar, He, clean dry air (CDA) and any combinations thereof.

In any preceding embodiments, the AD is carried out at a flow rate of a carrier gas of 180 to 2400 l/hr, preferably 300 to 1500 l/hr.

In any preceding embodiments, the temperature of the substrate during the deposition of the buffer layer and/or the biocompatible protective coating layer (BPCL) ranges from 5 °C to 50 °C, e.g., at least 15 °C, preferably not higher than 35°C.

### BRIEF DESCRIPTION OF THE DRAWINGS

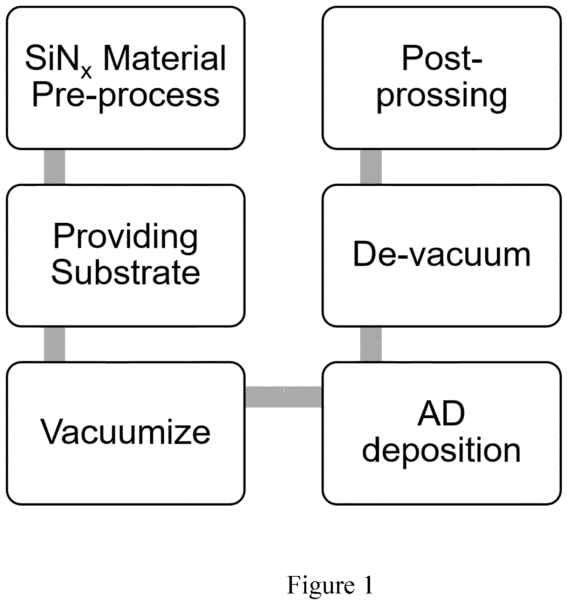

Figure 1 shows an illustrative scheme of the process of making the inventive medical implant.
Figure 2 shows exemplified articles (a knee joint and a screw).
Figures 3A to 3C show the microscopic behavior and characteristics of medical implants.
Figures 4A to 4D show optical or SEM images of medical implants upon wear tests.
Figures 5A to 5C show SEM images of the inventive screw upon wear tests.

### DETAILED DESCRIPTION OF THE INVENTION

In order to facilitate the understanding of the disclosure herein, terms as used herein are hereby defined below.

In the context of the specification and the claims, the singular forms "a", "an" and "the" include plural referents, unless specifically indicated otherwise. Unless otherwise stated, any and all examples or exemplary language (e.g., "such as") provided herein are merely used for better illustration of the present invention, instead of limiting the scope of the present invention.

It is to be understood that any numerical range recited in this specification is intended to include all sub-ranges encompassed therein. For example, a range from "50 to 70°C" includes all sub-ranges and specific values between the stated minimum value of 50°C and the stated maximum value of 70°C, inclusive, e.g., from 58°C to 67°C, and from 53°C to 62°C, 60°C or 68°C. Since the numerical ranges disclosed are continuous, they contain each numerical value between the minimum and maximum value. Unless otherwise specified, the various numerical ranges indicated in this specification are approximate.

In the present invention, the term "about" refers to an acceptable deviation of a given value measured by a person of ordinary skill in the art, depending, in part, on how to measure or determine the value.

In the present disclosure, the materials of a biocompatible protective coating layer include those having anti-wear/anti-abrading properties, including but are not limited to ceramics such as aluminum oxides, silicon nitrides, metal nitrides and any combinations thereof, e.g., silicon nitrides (e.g., SiNₓ, Si₃N₄), aluminum oxides (e.g., Al₂O₃), metal nitrides (e.g., TiN).

In the present disclosure, the term "silicon nitride" refers to a material having the formula of "SiNₓ" wherein x is a number within a reasonable range in view of the chemical bonding theory.

In the present disclosure, the term "biocompatible" or "biocompatibility" means having an ability to be in contact with a living system without producing an adverse effect, e.g., (severe) allergic response, damage to cells, tissues or organs in a living body, etc.

In the present disclosure, the term "porosity" refers to the level of pore space in a material.

In the present disclosure, the term "coefficient of kinetic friction" is the ratio of the friction force to the normal force experienced by a body moving on a dry, non-smooth surface, which may be, e.g., measured by Scratch tests.

In the present disclosure, the term "aerosol deposition" refers to a deposition process by which aerosol particles collect or deposit themselves on solid surfaces, decreasing the concentration of the particles in the air.

### Medical implant components

Medical implants may involve metals or alloys (such as CoCrMo, Ti-6Al-4V) or polymeric materials (such as PEEK, UHMWPE) as the substrate materials. Upon long-term use, bio-chemical corrosion and/or mechanical abrasion of the materials and stress shielding effect may result in adverse effects such as poisoning, allergic reaction, degeneration of peripheral tissues, etc. In addition, the hydrophobicity of certain substrate materials would negatively impact the prognosis.

To alleviate these disadvantages, certain materials such as alumina (Al₂O₃), Si₃N₄, titanium nitride (TiN) are believed to exhibit excellent mechanical properties, chemical inertness and biocompatibility, may be used as an anti-abrading layer or coating on the substrate materials. However, if the protective layer has a high porosity, weak adhesion and/or high surface roughness, it may easily shed, and particles thus produced may lead to dislocation or higher abrasion of countering parts of the implants.

Hence, in the present disclosure, the medical implant component comprises a biocompatible substrate and a biocompatible protective coating layer (BPCL) above or on the substrate. In one aspect, the medical implant component comprises a biocompatible substrate, a buffer layer on the substrate and a biocompatible protective coating layer (BPCL) on the buffer layer. The biocompatible protective coating layer (BPCL) has low porosity, high adhesion and low surface roughness. Details of the components of the medical implant component are described below.

### Biocompatible substrates

In the present disclosure, biocompatible substrates known in the art may be used once they have the desired characteristics, such as sufficient mechanical strength (hardness, robustness, etc.), chemical inertness, biocompatibility, etc. For example, the materials for making the biocompatible substrates may have a hardness of at least 400 HV (Vickers hardness) or at least a Shore D of 50 (Shore hardness). In various embodiments, the biocompatible substrate can be made from a material or materials selected from the group consisting of a biocompatible polymeric material, a biocompatible metal or alloy and a biocompatible ceramic.

Examples of the biocompatible polymeric material include, but are not limited to, polyketones such as polyetheretherketone (PEEK); (halogenated) polyalkylenes such as polyethylene (PE), poly(ethylene-propylene), polytetrafluoroethene (PTFE) and polyvinylchloride (PVC); polyurethanes, etc. In various embodiments, the PE is low-density polyethylene (LDPE), high-density polyethylene (HDPE) or ultra-high molecular weight polyethylene (UHMWPE).

Examples of the biocompatible metal or alloy include, but are not limited to, titanium (Ti) or an alloy thereof, zirconium (Zr) or an alloy thereof, tantalum (Ta) or an alloy thereof, niobium (Nb) or an alloy thereof, stainless steel, cobalt-chromium-molybdenum (Co-Cr-Mo) alloy, and Ti-6Al-4V alloy.

Examples of the biocompatible ceramic include, but are not limited to, oxide, carbide, nitride, or nitro-carbide of any of the following elements: silicon (Si), titanium (Ti), tantalum (Ta), tungsten (W), zirconium (Zr), niobium (Nb), chromium (Cr) and aluminum (Al).

In one embodiment, the biocompatible substrate has a surface roughness (Ra) of less than 0.3um; or higher than 0.01um. In one embodiment, the biocompatible substrate has a linear coefficient of thermal expansion ranging from 6*10⁻⁶ to 18*10⁻⁵.

### Biocompatible protective coating layer (BPCL)

In the present disclosure, the biocompatible protective coating layer (BPCL) is located on the substrate or above the substrate (e.g., when a buffer layer, described below, is present). The biocompatible protective coating layer (BPCL) shall have a porosity of less than 1.5%, preferably less than 1.3%, more preferably less than 1%, such that the medical implant may exhibit superior effect, e.g., good durability, anti-abrading effect, low friction, etc. The low porosity of this layer may be achieved by, e.g., the process of making the medical implant described herein.

In one embodiment, the biocompatible protective coating layer (BPCL) has a thickness of 0.5 µm to 70 µm, e.g., at least 0.5 µm, at least 1 µm, at least 2 µm, at least 3 µm, at least 4 µm, at least 5 µm, at least 6 µm, at least 7 µm, at least 8 µm, at least 9 µm, at least 10 µm, at least 11 µm, at least 12 µm, at least 13 µm, at least 14 µm, at least 15 µm, at least 20 µm, at least 25 µm, at least 30 µm, at least 35 µm; or at most 70 µm, at most 65 µm, at most 60 µm, at most 55 µm, at most 50 µm, at most 45 µm, at most 40 µm; or any reasonable numeric range constituted by the values noted above as the terminal point, e.g., 0.5 µm to 30 µm, 40 µm to 55 µm, 5 µm to 8 µm, etc.

In one embodiment, the biocompatible protective coating layer (BPCL) has a hardness (Vickers hardness) of 800 HV to 1400 HV, e.g., at least 850 HV, at least 900 HV, at least 950 HV, at least 1000 HV, at least 1050 HV, at least 1100 HV; or at most 1350 HV, at most 1300 HV, at most 1250 HV, at most 1200 HV, at most 1150 HV, at most 1100 HV; or any reasonable numeric range constituted by the values noted above as the terminal point, e.g., 900 HV to 1200 HV, 1150 HV to 1350 HV, 800 HV to 1000 HV, etc.

In one embodiment, the difference between the hardness of the material of the biocompatible substrate and biocompatible protective coating layer (BPCL) may be less than 900 HV, e.g., less than 800 HV, less than 700 HV, less than 600 HV, less than 500 HV, less than 400 HV, less than 300 HV, less than 200 HV, less than 100 HV, less than 50 HV, etc.

In one embodiment, biocompatible protective coating layer (BPCL) has a surface roughness of less than 0.25 µm, e.g., less than 0.2 µm, less than 0.15 µm, etc. Without being bound to the theory, when the surface roughness of the biocompatible protective coating layer (BPCL) is low, the friction between the medical implant and the contacting part may become low.

In one embodiment, the coefficient of kinetic friction between the medical implant component, in particular the BPCL, and the other corresponding medical component (such as a pad or insert) ranges from 0.08 to 0.7, e.g., less than 0.7, less than 0.65, less than 0.6, less than 0.55, less than 0.5, less than 0.45, less than 0.4, less than 0.3, less than 0.2, less than 0.1; or any reasonable numeric range constituted by the values noted above as the terminal point, e.g., 0.1 to 0.65, 0.1 to 0.55, etc.

The average molar ratio between the metal atoms and non-metal atoms of the biocompatible protective coating layer (BPCL) (i.e. Al/O, Si/N, Ti/N) may be in a reasonable range in view of the chemical bonding theory, the molar ratio deviates from perfect stoichiometry of material (e.g., about 0.67 for alumina, about 0.75 for silicon nitride, about 1 for titanium nitride, etc.) in the range of no more than 20%.

In one embodiment, the biocompatible protective coating layer (BPCL) is formed from particles having D50 ranging from 0.1 µm to 10 µm, e.g., being about 0.2 µm, about 0.3 µm, about 0.4 µm, about 0.5 µm, about 0.6 µm, about 0.7 µm, about 0.8 µm, about 0.9 µm, about 1 µm, about 1.5 µm, about 2 µm, about 2.5 µm, about 3 µm, about 3.5 µm, about 4 µm, about 4.5 µm, about 5 µm, about 5.5 µm, about 6 µm, about 6.5 µm, about 7 µm, about 8 µm, about 9 µm, about 10 µm; or any reasonable numeric range constituted by the values noted above as the terminal point, e.g., 0.5 µm to 3 µm, 0.8 µm to 5 µm, etc.

In one embodiment, the biocompatible protective coating layer (BPCL) has a linear coefficient of thermal expansion ranging from 3*10⁻⁶ °C⁻¹ to 15*10⁻⁶ °C⁻¹. In one embodiment, the difference between the biocompatible protective coating layer (BPCL) and the biocompatible substrate ranges from 1.1*10⁻⁶ to 18*10⁻⁵.

In one embodiment, the biocompatible protective coating layer (BPCL) has adhesion to the biocompatible substrate of at least 18 MPa, e.g., at least 19 MPa, at least 20 MPa, at least 21 MPa, at least 22 MPa, at least 23 MPa, at least 24 MPa, at least 25 MPa, at least 26 MPa, at least 27 MPa, at least 28 MPa; or any reasonable numeric range constituted by the values noted above as the terminal point, e.g., 20 MPa to 28 MPa, etc. The adhesion may be measured by Scratch tests, Rockwell C tests or ASTM-C1624.

### Buffer layer

In one aspect, the medical implant component further comprises a buffer layer, which is located on the substrate and between the biocompatible substrate and the biocompatible protective coating layer (BPCL). The buffer layer may assist the adhesion between the biocompatible substrate and the biocompatible protective coating layer (BPCL). In addition, the buffer layer may allow the surface to exhibit a lower roughness compared to the plain biocompatible substrate.

Examples of the materials for forming the buffer layer include, but are not limited to, oxide, nitride or carbide of any of the following elements: silicon (Si), aluminum (Al), zirconium (Zr) and boron (B).

In one embodiment, the buffer layer has a thickness of 0.5 µm to 10 µm, e.g., at least 0.5 µm, at least 0.75 µm, at least 1 µm, at least 1.5 µm, at least 2 µm, at least 2.5 µm, at least 3 µm, at least 3.5 µm, at least 4 µm, at least 4.5 µm, at least 5 µm; or at most 10 µm, at most 9.5 µm, at most 9 µm, at most 8.5 µm, at most 8 µm, at most 7.5 µm, at most 7 µm, at most 6.5 µm, at most 6 µm, at most 5.5 µm, at most 5 µm; or any reasonable numeric range constituted by the values noted above as the terminal point, e.g., 1 µm to 1.5 µm, 7 µm to 7.5 µm, 2 µm to 6 µm, etc.

In one embodiment, the buffer layer has adhesion to the biocompatible substrate of at least 18 MPa, e.g., at least 19 MPa, at least 20 MPa, at least 21 MPa, at least 22 MPa, at least 23 MPa, at least 24 MPa, at least 25 MPa, at least 26 MPa, at least 27 MPa, at least 28 MPa; or any reasonable numeric range constituted by the values noted above as the terminal point, e.g., 20 MPa to 28 MPa, etc. In one embodiment, the buffer layer has adhesion to the biocompatible protective coating layer (BPCL) of at least 18 MPa, e.g., at least 19 MPa, at least 20 MPa, at least 21 MPa, at least 22 MPa, at least 23 MPa, at least 24 MPa, at least 25 MPa, at least 26 MPa, at least 27 MPa, at least 28 MPa; or any reasonable numeric range constituted by the values noted above as the terminal point, e.g., 20 MPa to 28 MPa, etc. The adhesion may be measured by Scratch tests, Rockwell C tests or ASTM-C1624.

### Applications of medical implant components

Due to the excellent properties (including, but not limited to, good durability, good anti-abrasion, high density, low roughness, low porosity, good biocompatibility, etc.), the medical implant components can be used in various applications. Examples of the applications include, but are not limited to, a part of the entirety of an artificial joint (e.g., knee joint replacement, hip joint replacement, shoulder joint replacement, radiocarpal joint replacement; cups, heads, stems, etc.), an insert associated with an artificial joint, or a temporary anchorage device (e.g., used in orthodontics, surgery, etc., such as a medical locking plate).

### Process for making medical implant components

Conventional methods for making a coating or layer on the substrate of a medical implant component include plasma spraying, physical vapor deposition (PVD), chemical vapor deposition (CVD), spray processes (thermal or cold spray), sintering processes, etc. The inventors found that using aerosol deposition (AD) to deposit the specific ceramic material to form a biocompatible protective coating layer (BPCL) would be advantageous for prolonging life time of implant.

In brief, the AD process may exhibit the following advantages in making the medical implant component described herein: (1) the deposition rate is higher than that for PVD/CVD processes, which has the benefit of increasing the efficiency of production; (2) the temperature for conducting the process for depositing biocompatible protective coating layer (BPCL) can be significantly lower (e.g., near room temperature) than that for PVD/CVD (e.g., higher than 300 °C and even up to 800 °C), thermal spray or cold spray (around 300°C) processes; (3) the requirement of degree of vacuum is less restricted than that for conducting PVD/CVD processes; (4) the process may be more easily scaled up compared to PVD/CVD processes; (5) the thickness of the layer can be easily adjusted and may be larger; (6) the adhesion of the deposited layer is significantly higher than that obtained by PVD/CVD, thermal spray or cold spray processes; (7) the density and conformity of the deposited layer may be higher than that obtained by thermal spray or cold spray processes; (8) the deposited layer or coating may be a near-net-shape, which may be difficult when using PVD/CVD, thermal spray or cold spray processes; and (9) the costs may be significant lower than for PVD/CVD, thermal spray or cold spray processes.

In particular, coatings may be obtained by plasma spray processes, which are generally used in surface treatments; however, the porosity is too high, the adhesion may be weak and the surface roughness is also high. Sintered coatings may be dense and have a smooth surface; however, further processing, shaping and polishing may be difficult; additional problems, such as damage of the semi-products or heterogeneity in crystalline phases, may appear when scaling up the process in view of the variation of parameters of processing. PVD/CVD processes are sophisticated in the art and may provide coatings having good characteristics; however, the costs are high and there are inherent restrictions such as limited thickness of deposited layers (e.g., up to around 15 µm), deterioration of substrate materials due to high processing temperature, etc. In addition, the moderate adhesion of the coatings obtained by PVD/CVD processes may not be sufficient for preventing abrasion thereof, thereby leading to particles on the coating layers.

The inventors surprisingly found that AD processes are advantageous in making medical implant components, in particular the biocompatible protective coating layer (BPCL).

The present disclosure thus also relates to a process of making a medical implant component described herein, comprising the following steps:
(i) providing a biocompatible substrate in a deposition chamber;
(ii) reducing the pressure in the deposition chamber to less than 0.75 Torr; and
(iii) depositing a material of a biocompatible protective coating layer via aerosol deposition (AD) to form the biocompatible protective coating layer (BPCL) above or on the substrate.

In one aspect, the present disclosure relates to a process of making a medical implant component described herein, comprising the following steps:
(i) providing a biocompatible substrate in a deposition chamber;
(ii) reducing the pressure in the deposition chamber to less than 0.75 Torr;
(ii') depositing a buffer layer material selected from the group consisting of oxide, nitride or carbide of any of the following elements: silicon (Si), aluminum (Al), zirconium (Zr) and boron (B) to form a buffer layer on the substrate; and
(iii') depositing a material of a biocompatible protective coating layer via aerosol deposition (AD) to form the biocompatible protective coating layer (BPCL) on the buffer layer.

The biocompatible substrate may be those described herein. In one embodiment, the biocompatible substrate is made from a material or materials selected from the group consisting of a biocompatible polymeric material, a biocompatible metal or alloy and a biocompatible ceramic. In one embodiment, the biocompatible polymeric material is selected from the group consisting of polyetheretherketone (PEEK), polyethylene (PE) and polyvinylchloride (PVC). In one embodiment, the PE is low-density polyethylene (LDPE), high-density polyethylene (HDPE) or ultra-high molecular weight polyethylene (UHMWPE).

The AD processes can be conducted via known means and apparatuses. The apparatuses for conducing the AD process may include an aerosol generation unit (comprising, e.g., source(s) of carrier gas(es), a mass flow controller (MFC), an aerosol generating chamber), a deposition chamber (equipped with, e.g., a nozzle/nebulizer, a support plate or stage, etc.) and a vacuum system (e.g., a pump).

Various parameters of the AD processes may be adjusted. Examples of the parameters include, but are not limited to, the type of the carrier gas(es); the flow rate; the distance between the nozzle and the substrate support; the incident angle of the aerosol flow to the substrate; the degree of vacuum in the deposition chamber; the concentration, particle size, types of the material(s) of the a biocompatible protective coating layer in the aerosol flow; the temperature in the chamber; the temperature of the substrate; etc.

Examples of the carrier gas(es) include, but are not limited to, nitrogen (N₂), oxygen (O₂), argon (Ar), helium (He), clean dry air (CDA) and any combinations and proportions thereof. The flow rate of the carrier gas may be 180 to 2400l/hr, e.g., about 200 l/hr, about 250l/hr, about 300 l/hr, about 350 l/hr, about 400 l/hr, about 450 l/hr, about 500 l/hr, about 550 l/hr, about 600 l/hr, about 650 l/hr, about 700 l/hr, about 750 l/hr, about 800 l/hr, about 850l/hr, about 900 l/hr, about 950 l/hr, about 1000 l/hr, about 1100 l/hr, about 1200 l/hr, about 1300 l/hr, about 1400 l/hr, about 1500 l/hr, about 1600 l/hr, about 1700 l/hr, about 1800 l/hr, about 1900 l/hr, about 2000 l/hr, about 2100 l/hr, about 2200 l/hr, about 2300 l/hr; or any reasonable numeric range constituted by the values noted above as the terminal point, e.g., 500 l/hr to 650 l/hr, 300 to 1500 l/hr, etc.

The temperature (of biocompatible substrate) during the AD process may be controlled in the range from 5 °C to 50 °C, e.g., 15°C to 45°C, preferably no higher than 35 °C.

The material powders may have a D50 ranging from 0.5 µm to 10 µm, e.g., being about 0.2 µm, about 0.3 µm, about 0.4 µm, about 0.5 µm, about 0.6 µm, about 0.7 µm, about 0.8 µm, about 0.9 µm, about 1 µm, about 1.5 µm, about 2 µm, about 2.5 µm, about 3 µm, about 3.5 µm, about 4 µm, about 4.5 µm, about 5 µm, about 5.5 µm, about 6 µm, about 6.5 µm, about 7 µm, about 8 µm, about 9 µm, about 10 µm; or any reasonable numeric range constituted by the values noted above as the terminal point, e.g., 0.5 µm to 3 µm, 0.8 µm to 5 µm, etc. The raw material may be subject to pre-treatment(s), e.g., milling, sieving, etc., to provide the material(s) for the deposition via AD processes.

The buffer layer may be deposited via AD processes described herein, or physical vapor deposition (PVD) or chemical vapor deposition (CVD) processes. Examples of PVD/CVD processes include, but are not limited to, sputtering PVDs (with a DC or RF sputter), laser-assisted PVD (LA-PVD), low-pressure CVD (LPCVD), metal-organic CVD (MOCVD), plasma-enhanced CVD (PECVD), etc. Preferably, the buffer layer is produced via AD processes.

Once the deposition of the biocompatible protective coating layer (BPCL) is completed, the semi-product may be subject to post-processing, such as cleaning, shaping, etc.

### EXAMPLES

The following examples are provided to make the present invention more comprehensible to those of ordinary skill in the art to which the present invention pertains, but are not intended to limit the scope of the invention.

### Materials, Methodologies and Test Models

Materials of the biocompatible protective layer include Si₃N₄, Al₂O₃, TiN (4N purity) can be purchased from Sigma-Aldrich. CoCrMo alloy can be purchased from DePuy Synthes

Materials can be further treated via UPE- Celanese GUR@1020-E, if necessary.

Porosity of the layer can be measured by Hitachi S-4300 FE-SEM. Thickness and roughness can be measured by KLA-D500. Adhesion can be evaluated by Helmut-fisher Adhesion tester ST30 or Mitutoyo HR600 (Rockwell C indenter).

### Example 1

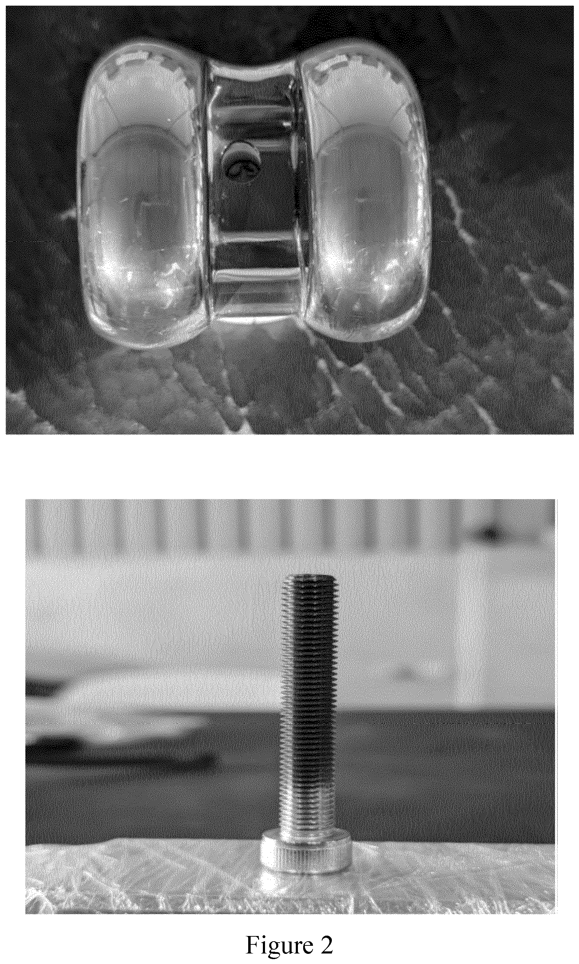

The CoCrMo alloy is used as the substrate material and is cleaned and washed to remove impurities, and then is dried before being placed on the support in the chamber of the AD apparatus. The temperature, degree of vacuum and other necessary parameters are set. The material powders (i.e. alumina, silicon nitride, titanium nitride) are loaded into an aerosol generating unit. Then, AD deposition of the materials is initiated. After deposition of the biocompatible protective coating layer (BPCL) is completed, the vacuum is broken and post-treatments are conducted on the semi-product to give the exemplified medical implant component. Pictures of the exemplified articles (a knee joint, a screw) were shown in Figure 2.

### Example 2

The CoCrMo alloy is used as the substrate material and is cleaned and washed to remove impurities, and then is dried before being placed on the support in the chamber of the AD apparatus. The temperature, degree of vacuum and other necessary parameters are set. In this case, biocompatible protective coating layer (BPCL) is formed by silicon nitride, and titanium nitride is used as the material for forming the buffer layer. The titanium nitride powders are loaded into separate aerosol generating units. Then, AD deposition of buffer layer materials is initiated. After deposition of the buffer layer is completed, AD deposition of silicon nitride is initiated. After deposition of the biocompatible protective coating layer (BPCL) is completed, the vacuum is broken and post-treatments are conducted on the semi-product to give the exemplified medical implant component.

### Example 3 (comparative)

A medical implant component is provided by using magnetron sputtering the silicon nitride layer on the CoCrMo substrate.

### Example 4 (comparative)

A medical implant component is provided by using magnetron sputtering the chromium nitride, as the buffer layer, on the CoCrMo substrate and the magnetron sputtering silicon nitride layer on the buffer layer.

### Example 5

Characteristics of the medical implant components set forth in Examples 1 to 4 are listed in Table 1:

**Table 1**

| **Characteristics** | **Ex. 1** | **Ex. 2** | **Ex. 3 (comparative)** | **Ex. 4 (comparative)** |
|---|---|---|---|---|
| porosity of protective coating layer | < 1%(1000X) (SiNₓ) | < 1%(1000X) (SiNₓ) | ∼ 1%(1000X) (SiNₓ) | ∼ 1%(1000X) (SiNₓ) |
| thickness of protective coating layer | 4.3 µm (SiNₓ) | 4.8 µm (SiNₓ) | 4.5 µm (SiNₓ) | 4.7 µm (SiNₓ) |
| surface roughness | ∼ 47.3 nm | ∼ 34.7 nm | ∼ 31.8 nm | ∼ 25.2 nm |
| adhesion between protective coating layer and buffer layer/substrate | HRC ISO Class 1 | HRC ISO Class 0 ∼ 1 | HRC ISO Class 6 | HRC ISO Class 1 |
| coefficient of kinetic friction (between the sample and a conventional insert) | 0.38 | 0.36 | 0.32 | 0.45 |
| specific wear rate (442Mpa) | 3.7*10⁻⁵ mm³/N | 3.3*10⁻⁵ mm³/N | 3.8*10⁻⁵ mm³/N | 3.5*10⁻⁵ mm³/N |
| Time for forming the layer (deposition rate) | about 205 s (21 nm/s) | about 229 s (21 nm/s) | about 2813 s (1.6 nm/s) | about 2938 s (1.6 nm/s) |

### Example 6

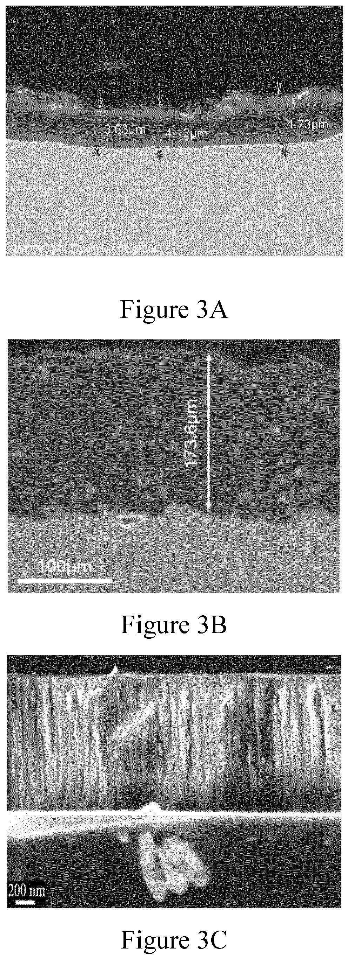

The microscopic behavior and characteristics were studied based on SEM technology. Figures 3A to 3C show the SEM images of the medical implant component as described in Example 1, a medical implant component with coating obtained via APS and a medical implant component with coating obtained via PVD. Apparently, the biocompatible protective coating layer (BPCL) were embedded into the substrate (or the buffer layer, if present), in particular forming chemical bonding, and the attached area therebetween significantly increased and thus the attachment was firm. In contrast, spaces or gaps may exhibit between a conventional coated layer (such as obtained via APS or PVD) and its substrate. Porosity existed in the coated layers (see Figure 3B), while no embedment of the coated layer to the substrate existed (see Figure 3C).

### Example 7

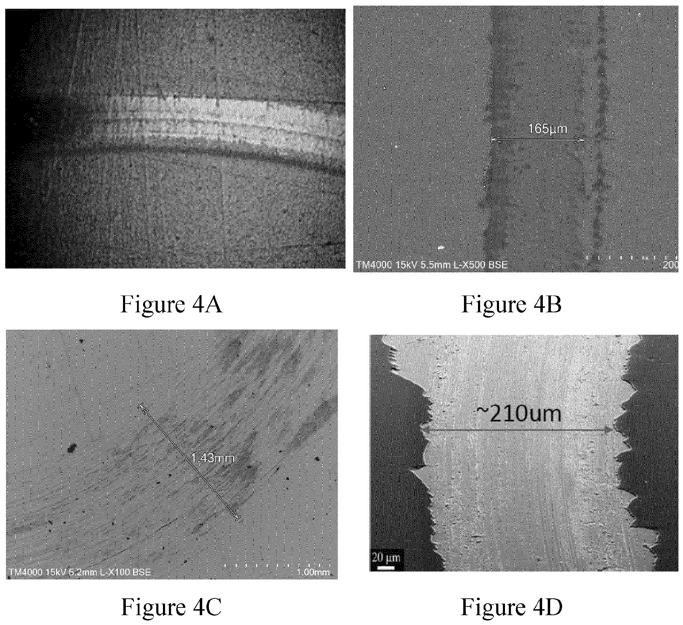

Wear tests were conducted on the medical implant component (a knee j oint) prepared based on the method described in Example 1 and conventional knee joints for comparison. were made based on Example 1 and 3 were manufactured. Wear testing conditions and results were conducted and summarized in Table 2 below. In particular, the articles were tested with an abraser running at a specific diameter for a designated distance, under a designated normal force. The abraded amounts were calculated based on the running diameter and abraded area. The optical image of the knee joint (Ex. 1) and the SEM images of the knee joint (Ex. 1), and the conventional knee joints (free of coating or coated via PVD) were shown in Figures 4A to 4D. Apparently, the inventive article exhibits superior characteristics (in particular anti-wearing characteristic) over conventional articles, e.g., significantly smaller abraded amounts under high normal force and running distance tests (see, Sample A vs. Sample B), higher wearing resistance (see, Sample A vs. Sample C, similar abraded amounts under harsher testing conditions).

**Table 2**

| Testing Sample No. | Abraser | Normal force | Running Distance | Diameter | Abraded amounts (mm³) |
|---|---|---|---|---|---|
| B knee joint (Ex. 1) | alumina | 5 N | 100 m | 5 mm | 0.002 |
| C conventional knee joint (no coating) | alumina | 5 N | 100 m | 5 mm | 0.095 |
| D conventional knee joint (coated via PVD) | alumina | 1 N | 3.925 m | 2.5 mm | 0.002 |

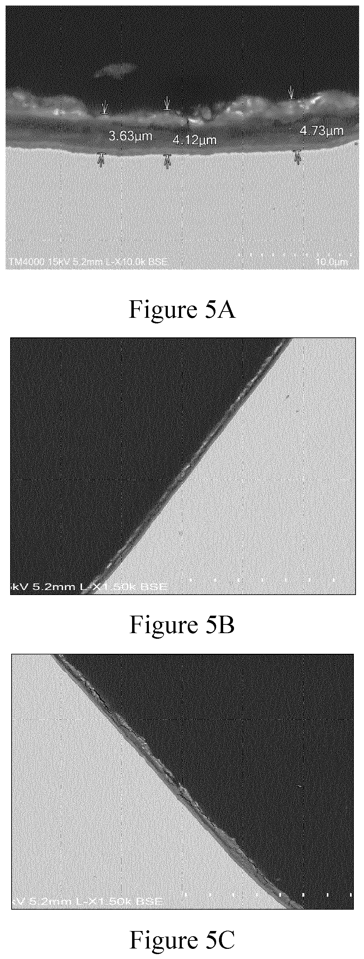

The SEM images of the bottom (Figure 5A) and the opposite sides (Figures 5B and 5C) of the screw after the abrasive tests were also provided, which apparently showed little abrasion of the coated layer after the wear test.

The invention apparently exhibits superior effects over the existing medical implant components. In particular, the adhesion between the biocompatible protective coating layer and the substrate is significantly enhanced. In addition, when using other deposition techniques (such as magnetron sputtering), the deposition rate may be limited, either by the nature of the techniques; even if a higher rate is used, the desired characteristics, e.g., low porosity, low surface roughness, high adhesion, etc., may not be provided. Without being bond to the theory, a higher depositing rate of materials via e.g., magnetron sputtering may lead to higher defect density, higher porosity, higher roughness, etc., of the deposited layer. In addition, when the depositing rate increases, the temperature of substrate subjected to the deposition may increase, which may lead to undesired effect or impact on the deposited layer.

A person of ordinary skill in the art of the subject invention should understand that variations and modification may be made to the teaching and the disclosure of the subject invention without departing from the spirit and scope of the subject application. Based on the contents above, the subject application intends to cover any variations and modifications thereof with the proviso that the variations or modifications or their equivalents fall within the scope as defined in the appended claims.

## Claims

1. A medical implant component, comprising:
(a) a biocompatible substrate, and
(b) a biocompatible protective coating layer (BPCL) above or on the substrate;
wherein the biocompatible substrate is made from a material or materials selected from the group consisting of a biocompatible polymeric material, a biocompatible metal or alloy and a biocompatible ceramic, and
wherein the biocompatible protective coating layer (BPCL) has a porosity of less than 1.5%.

2. The medical implant component according to Claim 1, wherein the biocompatible protective coating layer (BPCL) is formed by a material or materials selected from the group consisting of aluminum oxides, silicon nitrides, metal nitrides and any combinations thereof.

3. The medical implant component according to any preceding claims, wherein the biocompatible protective coating layer (BPCL) has one or more of the following characteristics: (a) a thickness of 0.5 µm to 70 µm; (b) a hardness (Vickers hardness) of 800 HV to 1400 HV; (c) a surface roughness of less than 0.25 µm; (d) the stoichiometry change in the atoms of materials of biocompatible protective coating layer (BPCL) of smaller than 20%; and (e) being formed from particles having a D50 ranging from 0.1 µm to 10 µm.

4. The medical implant component according to any preceding claims, wherein the biocompatible polymeric material is selected from the group consisting of polyketones, (halogenated) polyalkylenes and polyurethanes, preferably selected from the group consisting of polyetheretherketone (PEEK), polyethylene (PE), polytetrafluoroethene (PTFE), polyvinylchloride (PVC) and polyurethane (PU), more preferably the PE being low-density polyethylene (LDPE), high-density polyethylene (HDPE) or ultra-high molecular weight polyethylene (UHMWPE).

5. The medical implant component according to any preceding claims, wherein the biocompatible polymeric material has a hardness of at least 400 HV (Vickers hardness) or at least a Shore D of 50 (Shore hardness).

6. The medical implant component according to any one of Claims 1-3, wherein the biocompatible metal or alloy is selected from the group consisting of titanium (Ti) or an alloy thereof, zirconium (Zr) or an alloy thereof, tantalum (Ta) or an alloy thereof, niobium (Nb) or an alloy thereof, stainless steel, cobalt-chromium-molybdenum (Co-Cr-Mo) alloy, and Ti-6Al-4V alloy; or the biocompatible ceramic is selected from the group consisting of oxide, carbide, nitride, or nitro-carbide of any of the following elements: silicon (Si), titanium (Ti), tantalum (Ta), tungsten (W), zirconium (Zr), niobium (Nb), chromium (Cr) and aluminum (Al).

7. The medical implant component according to any preceding claims, further comprising a buffer layer, wherein the buffer layer is located between the biocompatible substrate and the biocompatible protective coating layer (BPCL), and wherein the buffer layer is made from oxide, nitride or carbide of any of the following elements: silicon (Si), aluminum (Al), zirconium (Zr) and boron (B).

8. The medical implant component according to Claim 7, wherein the buffer layer has a thickness of 0.5 µm to 10 µm.

9. The medical implant component according to Claim 7 or 8, wherein the adhesion between the buffer layer and the substrate is at least 18 Mpa.

10. The medical implant component according to any preceding claims, which is a part or the entirety of an artificial j oint, an insert associated with an artificial j oint or a temporary anchorage device.

11. A process of making a medical implant component according to any preceding claims, comprising the following steps:
(i) providing a biocompatible substrate in a deposition chamber;
(ii) reducing the pressure in the deposition chamber to less than 0.75 Torr; and
(iii) depositing a material of a biocompatible protective coating layer (BPCL) via aerosol deposition (AD) to form the biocompatible protective coating layer (BPCL) above or on the substrate, preferably the temperature of the substrate ranging from 5 °C to 50 °C.

12. The process according to Claim 11, further comprising a step (ii') following the step (ii):
(ii') depositing a buffer layer material selected from the group consisting of oxide, nitride or carbide of any of the following elements: silicon (Si), aluminum (Al), zirconium (Zr) and boron (B) to form a buffer layer on the substrate, preferably the temperature of the substrate ranging from 5 °C to 50 °C, and
wherein the step (ii) is step (ii'):
(iii') depositing a material of a biocompatible protective coating layer (BPCL) via aerosol deposition (AD) to form the biocompatible protective coating layer(BPCL) above or on the buffer layer.

13. The process according to Claim 12, wherein the deposition of the buffer layer is achieved via AD, physical vapor deposition (PVD) or chemical vapor deposition (CVD).

14. The process according to any of Claims 11 to 13, wherein the AD is carried out with a carrier gas selected from the group consisting of N₂, O₂, Ar, He, clean dry air (CDA) and any combinations thereof, preferably carried out at a flow rate of a carrier gas of 180 to 2400 l/hr.

15. A medical implant component, **characterized in that** it comprises a biocompatible protective coating layer (BPCL) and optionally a buffer layer, wherein the biocompatible protective coating layer (BPCL) has a porosity of less than 1.5%.
